(19) Europäisches Patentamt
European Patent Office
Office européen des brevets

(11) Veröffentlichungsnummer: **0 345 572**
**A2**

(12) # EUROPÄISCHE PATENTANMELDUNG

(21) Anmeldenummer: **89109590.3**

(22) Anmeldetag: **27.05.89**

(51) Int. Cl.4: **C07C 143/78 , C07C 143/75 , C07C 151/00 , C07C 131/00 , C07F 9/38 , A61K 31/18**

(30) Priorität: **04.06.88 DE 3819052**

(43) Veröffentlichungstag der Anmeldung: **13.12.89 Patentblatt 89/50**

(84) Benannte Vertragsstaaten: **AT BE CH DE FR GB IT LI NL SE**

(71) Anmelder: **BASF Aktiengesellschaft**
**Carl-Bosch-Strasse 38**
**D-6700 Ludwigshafen(DE)**

(72) Erfinder: **Schmied, Bernhard, Dr.**
**Taunusstrasse 20**
**D-6710 Frankenthal(DE)**
Erfinder: **Lehmann, Hans Dieter, Prof. Dr.**
**Im Hefen 15**
**D-6945 Hirschberg(DE)**
Erfinder: **Baldinger, Verena, Dr.**
**Schiffsgasse 6**
**D-6900 Heidelberg(DE)**
Erfinder: **Ruebsamen, Klaus, Dr.**
**Erschigweg 19**
**D-6730 Neustadt(DE)**

(54) **Neue Sulfonamid-Derivate, ihre Herstellung und Arzneimittel daraus.**

(57) Sulfonamid-Derivate der Formel I

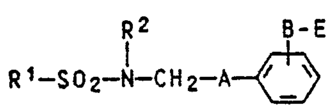

I

in der
$R^1$ eine $C_{1-4}$-Alkyl- oder Phenylgruppe bedeutet, die durch Halogenatome, Trifluoromethyl- oder $C_{1-4}$-Alkylgruppen substituiert sein kann,
$R^2$ ein Wasserstoffatom oder eine $C_{1-4}$-Alkylgruppe bedeutet,
A für eine der Gruppen $CHOR^3$, C=O, C=S oder $C=N-OR^4$ steht, wobei $R^3$ ein Wasserstoffatom, eine $C_{1-4}$-Alkyl-, Phenyl-, Benzyl- oder $C_{1-4}$-Acylgruppe und
$R^4$ ein Wasserstoffatom oder eine Methylgruppe bedeutet,
B eine m- oder p-ständige Methylen-, Ethylen- oder, wenn A nicht für eine Carbonylgruppe steht, auch eine Methylenoxy-Gruppe bedeuten kann, wobei das Sauerstoffatom an den Aromaten gebunden ist,
E einen Carboxyl-, Phosphonsäure- oder Sulfonsäurerest, deren physiologisch unbedenkliche Salze, Ester mit $C_{1-4}$-Alkoholen und Amide, sowie den Tetrazol-5-yl-Rest bedeuten kann,
deren Herstellung und Verwendung als Arzneimittel.

EP 0 345 572 A2

Xerox Copy Centre

## Neue Sulfonamid-Derivate, ihre Herstellung und Arzneimittel daraus

Die Erfindung betrifft neue substituierte Sulfonamide, Verfahren zu deren Herstellung sowie daraus hergestellte Arzneimittel.

Substituierte Sulfonamide mit pharmakologischen Wirkungen sind seit langer Zeit bekannt (z. B.: J. Prakt. Chem. 77, 369 (1908); J. Pharm. Soc. Japan 70, 283 (1950)). Auch eine Reihe von Sulfonamiden mit lipidsenkender und thrombocytenaggregationshemmender Wirkung sind bereits beschrieben (z. B.: DE-OS 2809 377, DE-OS 3000 377 und Thromb. Res. 33, 277 (1984)).

Es wurde nun gefunden, daß sich die neuen Sulfonamid-Derivate der Formel I

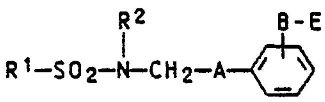

I

in der

$R^1$ eine $C_{1-4}$-Alkyl- oder Phenylgruppe bedeutet, die durch Halogenatome, Trifluoromethyl- oder $C_{1-4}$-Alkylgruppen substituiert sein kann,

$R^2$ ein Wasserstoffatom oder eine $C_{1-4}$-Alkylgruppe bedeutet,

A für eine der Gruppen $CHOR^3$, C=O, C=S oder $C=N-OR^4$ steht, wobei $R^3$ ein Wasserstoffatom, eine $C_{1-4}$-Alkyl-, Phenyl-, Benzyl- oder $C_{1-4}$-Acrylgruppe und $R^4$ ein Wasserstoffatom oder eine Methylgruppe bedeutet,

B eine m- oder p-ständige Methylen-, Ethylen- oder, wenn A nicht für eine Carbonylgruppe steht, auch eine Methylenoxy-Gruppe bedeuten kann, wobei das Sauerstoffatom an den Aromaten gebunden ist,

E einen Carboxyl-, Phosphonsäure- oder Sulfonsäurerest, deren physiologisch unbedenkliche Salze, Ester mit $C_{1-4}$-Alkoholen und Amide, sowie den Tetrazol-5-yl-Rest bedeuten kann,

bei guter oraler Verfügbarkeit durch eine sehr hohe und langanhaltende thrombocytenaggregationshemmende Wirkung auszeichnen.

Besonders bevorzugt sind die folgenden Verbindungen:

4-(1-Oxo-2-phenylsulfonamido-ethyl)phenylessigsäure
3-(1-Oxo-2-phenylsulfonamido-ethyl)phenylessigsäure
4-[1-Oxo-2-(4-chlor-phenylsulfonamido)ethyl]phenylessigsäure
3-[1-Oxo-2-(4-chlor-phenylsulfonamido)ethyl]phenylessigsäure
3-(4-[1-Oxo-2-(phenylsulfonamido)ethyl]phenyl)propionsäure
3-(4-[1-Thiono-2-(phenylsulfonamido)ethyl]phenyl)propionsäure
3-(4-[1-Oximino-2-(phenylsulfonamido)ethyl]phenyl)propionsäure
3-(4-[1-Methoximino-2-(phenylsulfonamido)ethyl]phenyl)propionsäure
3-(4-[1-Oxo-2-(4-methyl-phenylsulfonamido)-ethyl]phenyl)propionsäure
3-(4-[1-Oxo-2-(3,4-dimethyl-phenylsulfonamido)-ethyl]phenyl)propionsäure
3-(4-[1-Oxo-2-(2,4,6-trimethyl-phenylsulfonamido)-ethyl]phenyl)propionsäure
3-(4-[1-Oxo-2-(4-ethyl-phenylsulfonamido)-ethyl]phenyl)propionsäure
3-(4-[1-Oxo-2-(4-methoxy-phenylsulfonamido)-ethyl]phenyl)propionsäure
3-(4-[1-Oxo-2-(4-trifluoromethyl-phenylsulfonamido)-ethyl]phenyl)propionsäure
3-(4-[1-Oxo-2-(4-fluor-phenylsulfonamido)-ethyl]phenyl)propionsäure
3-(4-[1-Oxo-2-(2-chlor-phenylsulfonamido)-ethyl]phenyl)propionsäure
3-(4-[1-Oxo-2-(2,6-dichlor-phenylsulfonamido)-ethyl]phenyl)propionsäure
3-(4-[1-Oxo-2-(4-chlor-phenylsulfonamido)-ethyl]phenyl)propionsäure
3-(4-[1-Thiono-2-(4-chlor-phenylsulfonamido)-ethyl]phenyl)propionsäure
3-(4-[1-Oximino-2-(4-chlor-phenylsulfonamido)-ethyl]phenyl)propionsäure
3-(4-[1-Methoximino-2-(4-chlor-phenylsulfonamido)-ethyl]phenyl)propionsäure
4-(1-Hydroxy-2-phenylsulfonamido-ethyl)phenylessigsäure
3-(1-Hydroxy-2-phenylsulfonamido-ethyl)phenylessigsäure
4-[1-Hydroxy-2-(4-chlor-phenylsulfonamido)ethyl]phenylessigsäure
3-[1-Hydroxy-2-(4-chlor-phenylsulfonamido)ethyl]phenylessigsäure
3-(4-[1-Hydroxy-2-(phenylsulfonamido)ethyl]phenyl-propionsäure
3-(4-[1-Hydroxy-2-(4-chlor-phenylsulfonamido-ethyl]phenylpropionsäure
4-(1-Hydroxy-2-phenylsulfonamido-ethyl)phenoxyessigsäure

4-[1-Hydroxy-2-(4-chlor-phenylsulfonamidoethyl]phenoxyessigsäure
4-[1-Hydroxy-2-(4-fluor-phenylsulfonamidoethyl]phenoxyessigsäure
4-[1-Hydroxy-2-(4-chlor-phenylsulfonamidoethyl]phenoxymethansulfonsäure
4-[1-Hydroxy-2-(4-chlor-phenylsulfonamidoethyl]phenoxymethanphosphonsäure
4-(1-Methoxy-2-phenylsulfonamido-ethyl)phenylessigsäure
3-[1-Methoxy-2-phenylsulfonamido-ethyl)phenylessigsäure
4-[1-Methoxy-2-(4-chlor-phenylsulfonamido)ethyl]phenylessigsäure
(+)-4-[1-Methoxy-2-(4-chlor-phenylsulfonamido)ethyl]phenylessigsäure
(-)-4-[1-Methoxy-2-(4-chlor-phenylsulfonamido)ethyl]phenylessigsäure
3-[1-Methoxy-2-(4-chlor-phenylsulfonamido)ethyl]phenylessigsäure
3-(4-[1-Methoxy-2-phenylsulfonamido-ethyl]phenyl)propionsäure 3-(4-[1-Methoxy-2-(4-chlor-phenylsulfonamido)ethyl]phenyl)propionsäure
4-(1-Methoxy-2-phenylsulfonamido-ethyl)phenoxyessigsäure
4-[1-Methoxy-2-(4-fluor-phenylsulfonamidoethyl]phenylessigsäure
4-[1-Methoxy-2-(4-chlor-phenylsulfonamidoethyl]phenoxyessigsäure
4-[1-Methoxy-2-(4-chlor-phenylsulfonamidoethyl]phenoxyessigsäure
(+)-4-[1-Methoxy-2-(4-chlor-phenylsulfonamido)ethyl]phenoxyessigsäure
(-)-4-[1-Methoxy-2-(4-chlor-phenylsulfonamido)ethyl]phenoxyessigsäure
4-(1-Acetoxy-2-phenylsulfonamido-ethyl)phenoxyessigsäure
4-[1-Acetoxy-2-(4-chlor-phenylsulfonamido)ethyl]phenoxyessigsäure
4-(1-Ethoxy-2-phenylsulfonamido-ethyl)phenoxyessigsäure
4-[1-Methoxy-2-(N-phenylsulfonyl, N-methylamino)ethyl]phenoxyessigsäure

Die neuen Verbindungen der Formel I, werden hergestellt indem man
a) Verbindungen der Formel II,

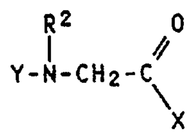

II

in der
$R^2$ ein Wasserstoffatom oder eine $C_{1-4}$-Alkylgruppe, bevorzugt ein Wasserstoffatom oder eine Methylgruppe bedeutet,
X eine Abgangsgruppe darstellt, bevorzugt ein Chlor-, Brom- oder Jodatom, und
Y für eine hydrolytisch oder hydrogenolytisch abspaltbare Schutzgruppe oder den Rest $R^1$, mit der in Formel I beschriebenen Bedeutung steht, bevorzugt für eine Acetyl-, Trifluoroacetyl-, Benzyloxycarbonyl-, Phenylsulfonyl- oder (unter Einbeziehung von $NR^2$) eine Phthalimido-Gruppe, umsetzt mit einer Verbindung der Formel III

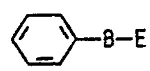

III.

in der B und E die für Formel I beschriebenen Bedeutungen besitzen, für E aber bevorzugt der Carboxylsowie der Methoxy- oder Ethoxycarbonylrest steht,
und von den so erhaltenen Verbindungen gegebenenfalls die Schutzgruppen abspaltet und mit Verbindungen der Formel IV,
$R^1$-$SO_2$-X IV,
in der $R^1$ die für Formel I beschriebene Bedeutung zukommt und
X eine Abgangsgruppe darstellt, bevorzugt ein Chlor-, Brom, Jodatom oder eine Alkoxygruppe, umsetzt, gegebenenfalls die Carbonylgruppe zum Alkohol reduziert und nach bekannten Methoden, wie sie im experimentellen Teil beispielhaft beschrieben sind, in die gewünschten Derivate überführt.

Die oben beschriebenen Reaktionen, Acylierung, Schutzgruppenabspaltung, Einführung des Restes $R^1$-$SO_2$-, Reduktion und Derivatisierung können auch in jeder anderen sinnvollen Reihenfolge durchgeführt werden oder teilweise entfallen.

Die Friedel-Crafts-Acylierung wird in Gegenwart einer Lewis-Säure, bevorzugt Aluminiumchlorid, Zinkchlorid oder Bortrifluorid, in einem inerten Lösungsmittel, bevorzugt Methylenchlorid, Dichlorethan, Nitrobenzol, Dimethylformamid oder Gemischen der vorgenannten Lösungsmittel, bei Temperaturen zwischen

0° C und 160° C, bevorzugt zwischen Raumtemperatur und 80° C, vorgenommen.

Die Schutzgruppenabspaltung wird nach üblichen Methoden, bevorzugt mit Salzsäure, Schwefelsäure oder Alkalihydroxid in Wasser oder in Gemischen aus Wasser und Isopropanol, Tetrahydrofuran oder Dioxan bei Temperaturen zwischen 0 und 100° C, für den Benzyloxycarbonylrest dagegen bevorzugt bei 0° C bis Raumtemperatur mit Wasserstoff in Gegenwart eines Katalysators wie Palladium/Kohle in einem Lösungsmittel wie Wasser, Eisessig, Methanol, Ethanol, Isopropanol oder Essigsäureethylester durchgeführt.

Die Sulfonierung wird ebenfalls nach üblichen Methoden in einem inerten Lösungsmittel, bevorzugt Wasser, Methanol, Ethanol, Isopropanol, Dimethylformamid, Dimethylsulfoxid, Pyridin, Tetrahydrofuran oder Gemischen dieses Lösungsmittels unter Zusatz einer Base, bevorzugt Soda, Pottasche, Bicarbonat, Alkalihydroxid, Triethylamin oder Pyridin, bei Temperaturen zwischen 0 und 160° C, bevorzugt zwischen 0 und 70° C, vorgenommen, und die Reduktion der Carbonylgruppe zum Alkohol wird mit Metallhydriden oder katalytisch erregtem Wasserstoff, bevorzugt jedoch bei 0° C bis Raumtemperatur mit Natriumborhydrid in Wasser, Methanol, Isopropanol, Acetonitril oder mit Wasserstoff in Gegenwart von Palladium/Kohle in einem Lösungsmittel wie Wasser, Eisessig, Methanol, Ethanol, Isopropanol oder Essigsäureethylester durchgeführt.

Die Verbindungen der Formel I lassen sich weiter herstellen, indem man

b) Octopamin umsetzt mit Verbindungen der Formel IV

$R^1$-$SO_2$-X IV,

in der $R^1$ die in Formel I beschriebene Bedeutung zukommt und

X eine Abgangsgruppe darstellt, bevorzugt Chlor-, Brom-, Jodatome oder eine Alkoxyrupppe, das Reaktionsprodukt am phenolischen Sauerstoff, gegebenenfalls unter Aktivierung mit Trimethylsilyl-chlorid, mit einer Verbindung der Formel V

X-$CH_2$-E-Y V,

in der X eine Abgangsgruppe darstellt, bevorzugt ein Chlor-, Brom-, Jodatom, den Tosylat-, Mesylat- oder Triflatrest,

E die in Formel I beschriebene Bedeutung besitzt, alkyliert, die Schutzgruppen abspaltet und nach bekannten Methoden, wie sie im experimentellen Teil beispielhaft beschrieben sind, in Derivate der aliphatischen Hydroxygruppe überführt.

Die vorstehend genannten Reaktionen, Sulfonierung, Aktivierung, Alkylierung, Schutzgruppenabspaltung und Derivatisierung können auch in jeder anderen sinnvollen Reihenfolge durchgeführt werden oder teilweise entfallen.

Die Sulfonierung wird auch hier nach üblichen Methoden, vorzugsweise wie weiter oben beschrieben, vorgenommen. Die Alkylierung am phenolischen Sauerstoff wird in einem inerten Lösungsmittel, bevorzugt Wasser, Methanol, Ethanol, Isopropanol, Dimethylformamid, Dimethylsulfoxid, Tetrahydrofuran, Pyridin oder in Gemischen der genannten Lösungsmittel unter Zusatz einer Base, bevorzugt Alkalihydroxid, Soda, Pottasche, Cesiumcarbonat, Ammoniumhydroxid, Triethylamin oder nach Isolierung des Phenolates, bevorzugt des Cäsium-Phenolates, in einem der genannten Lösungsmittel bei Temperaturen zwischen 0 und 160° C, bevorzugt zwischen 0 und 70° C, durchgeführt.

Alle Verbindungen der Formel I, in denen A für $CHOR^3$ steht, besitzen ein chirales Kohlenstoffatom. Folglich können diese Verbindungen entweder in optisch aktiven Formen oder als racemische Mischung hergestellt werden.

Wenn erwünscht, können die Racemate der Formel I, in denen A für $CHOR^3$ steht, und E einen Carboxyl- oder Sulfonsäurerest darstellt, durch Umsetzung mit chiralen Aminen in diastereomere Salze überführt werden. Beispiele für chirale Amine sind die optisch aktiven Formen von Dehydroabietylamin, Brucin, Ephedrin, Cinchonin, α-Methylbenzylamin, 2-Aminobutanol, 1-Phenylethylamin, 1-Cyclohexylethylamin und 3-Aminomethylpinan.

Die racemische Vorstufe VI

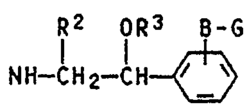

VI

in der $R^2$, $R^3$ und B die in Formel I beschriebenen Bedeutungen besitzen und G für eine Tetrazol-5-yl-Gruppe oder für das Amid oder den $C_{1-4}$-Alkylester einer Carboxylgruppe steht, kann durch Umsetzung mit chiralen Säuren ebenfalls in diastereomere Salze überführt werden. Beispiele für chirale Säuren sind die optisch aktiven Formen der Campher-10-sulfonsäure, α-Bromcampher-π-sulfonsäure, Camphersäure, Menthoxyessigsäure, Weinsäure, Mandelsäure, 0,0-Diacetyl-, 0,0-Dibenzoyl- oder 0,0-Di-4-toluylweinsäure und Diacetonketogulonsäure.

Die Racemate können in Form ihrer diastereomeren Salze durch herkömmliche Trenntechniken, z. B. durch fraktionierte Kristallisation oder Säulenchromatographie, in ihre optischen Antipoden gespalten werden.

Die erfindungsgemäßen Verbindungen besitzen wertvolle pharmakologische Eigenschaften, insbesondere eine ausgeprägte thrombozytenaggregationshemmende Wirkung bei guter oraler Verfügbarkeit. Außerdem gibt es Hinweise auf eine Hemmwirkung bei der Tumormetastasierung und der Arteriosklerose.

Die neuen Verbindungen können in üblicher Weise oral oder parenteral (subkutan, intravenös, intramuskulär, intraperitoneal) verabfolgt werden.

Die Dosierung hängt vom Alter, Zustand und Gewicht des Patienten sowie von der Applikationsform ab. In der Regel beträgt die tägliche Wirkstoffdosis von 0,1 bis 10, vorzugsweise 0,5 bis 5 mg bei parenteraler und 1 bis 100, vorzugsweise 5 bis 50 mg bei oraler Anwendung pro Patient und Tag.

Die neuen Verbindungen können in den gebräuchlichen galenischen Applikationsformen fest oder flüssig angewendet werden, z. B. als Lösungen oder Suppositorien. Diese werden in üblicher Weise hergestellt. Die Wirkstoffe können dabei mit den gebräuchlichen galenischen Hilfsmitteln, wie Tablettenbindern, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln und/oder Antioxidantien verarbeitet werden (vgl. H. Sucher et al.: Pharmazeutische Technologie, Thieme-Verlag, Stuttgart 1978). Die so erhaltenen Zubereitungen enthalten den Wirkstoff normalerweise in einer Menge von 0,1 bis 99 Gew.-%.

Syntheseplan am Beispiel der erfindungsgemäßen Verbindungen 9a, 16a und 17a

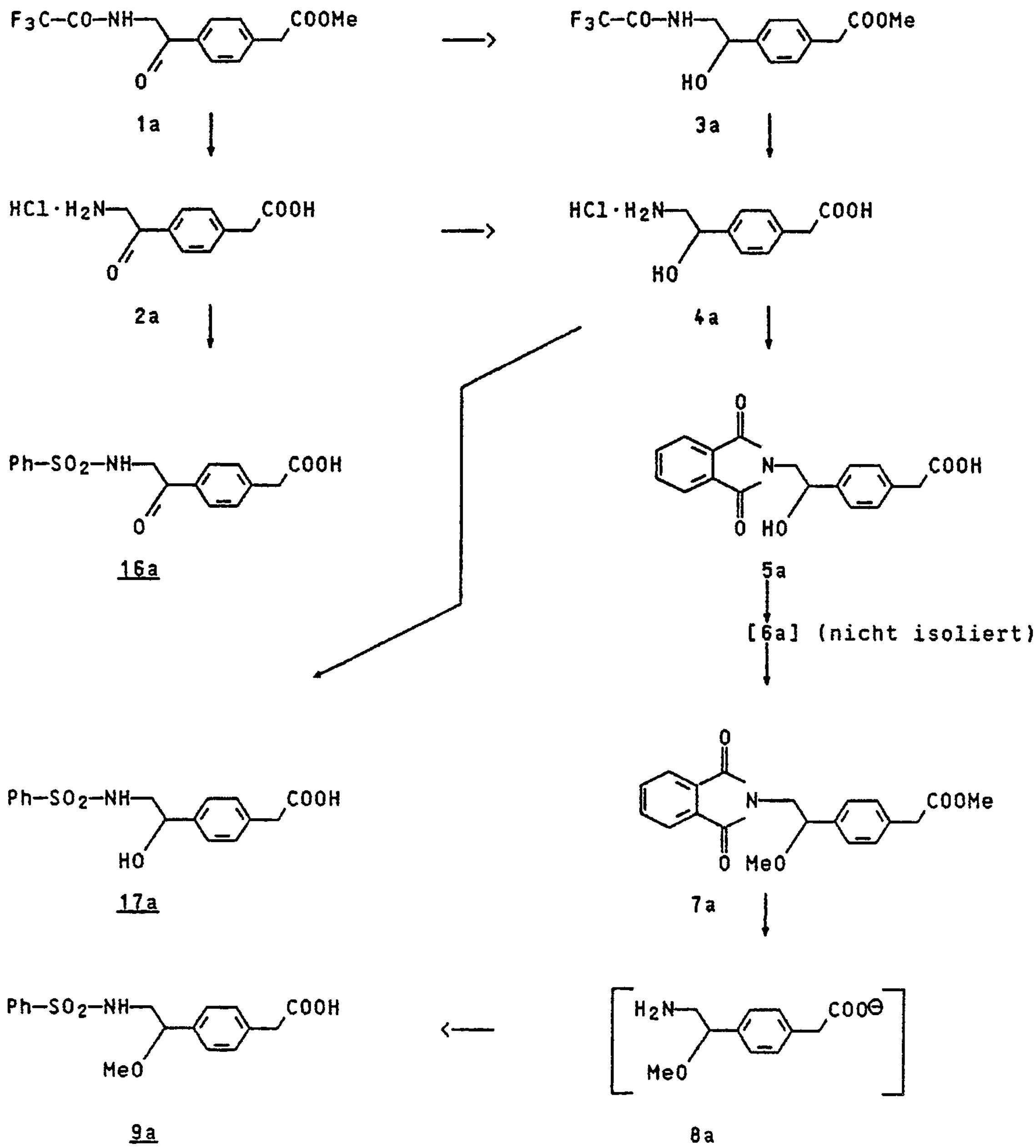


1a 3a 2a 4a 16a 5a 17a 7a 9a 8a

Syntheseplan am Beispiel der erfindungsgemäßen Verbindungen 9d, 12a, 13a und 15

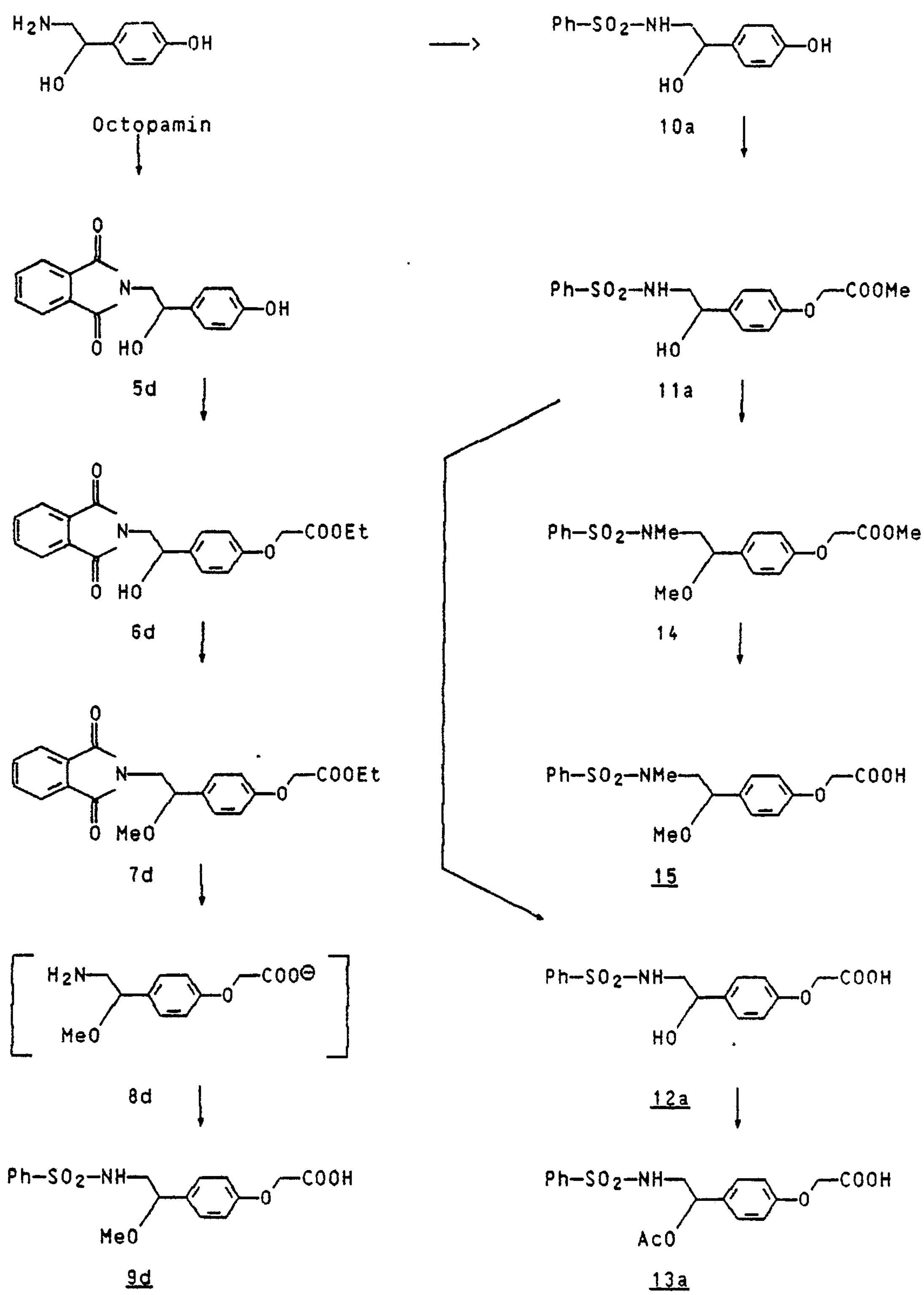

Octopamin

10a

5d

11a

6d

14

7d

15

8d

12a

9d

13a

Experimenteller Teil

Der Verlauf aller Reaktionen wurde mittels Dünnschichtchromatographie auf Kieselgelfertigplatten (Kieselgel 60 $F_{254}$, 5 x 10 cm, Merck Nr. 5789) verfolgt. Als Fließmittel wurden in Abhängigkeit von der Polarität der untersuchten Verbindungen folgende Lösungsmittelgemische benutzt:

(A) Dichlormethan : Methanol (40 : 1)

(B) Dichlormethan : Methanol : Eisessig (20 : 2 : 1)

(C) n-Butanol : Essigsäureethylester : $H_2O$ (6 : 2 : 2)

(D) n-Butanol : Essigsäureethylester : $H_2O$ : Pyridin (60 : 6 : 24 : 20)

Die Produkte aller nachfolgend beschriebenen Reaktionen wurden $^1$H-NMR-spektroskopisch untersucht und stehen im Einklang mit der angegebenen Struktur.

Herstellung der Vorprodukte

3- und 4-(1-Oxo-2-trifluoroacetamido-ethyl)phenylessigsäuremethylester (1a, 1b)

In 715 g Aluminiumtrichlorid wurden langsam 196 g Dimethylformamid eingetropft. Nachdem die stark exotherme Reaktion abgeklungen war, wurde bei 80° C unter Rühren langsam eine Lösung von 1,08 mol N-(Trifluoroacetyl)glycylchlorid (J.E. Nordlander et.al. J. Org. Chem. 49, 4107 (1984) in 1,7 l Dichlormethan und 6 ml Pyridin zugetropft. Nachdem die Gasentwicklung abgeklungen war, wurden bei 70 - 80° C 161 g Phenylessigsäuremethylester eingetropft und die Mischung bei der selben Temperatur noch weitere 30 min gerührt.

Das Reaktionsgemisch wurde in 6 l Eiswasser eingegossen, mit konz. Salzsäure angesäuert und das ausgefallene Produktgemisch abfiltriert. Rohausbeute: ca. 281 g kristalliner Feststoff.

Das Rohprodukt wurde 3 mal mit je 150 ml Diethylether bei Raumtemperatur digeriert. Der kristalline Rückstand wurde filtriert, mit ca. 50 ml Diethylether nachgewaschen und im Hochvakuum getrocknet. Ausbeute: 55 g (17 %) 4-(1-Oxo-2-trifluoroacetamido-ethyl)phenylessigsäuremethylester (1a); Schmp. 131 - 132° C.

Die vereinigten Etherfiltrate wurden im Vakuum bis zur Trockene eingeengt und der dunkle Rückstand (226 g) durch Flash-Chromatographie (1 kg Kieselgel 60, mesh 230, (Merck 7729); Säule 160 x 190 mm; Gradientenelution: 100 % Cyclohexan bis 80 % Cyclohexan/20 % Essigsäureethylester; Wasserstrahlvakuum) von den gefärbten Verunreinigungen befreit. Mit 80 % Cyclohexan/20 % Essigsäureethylester wurden 113 g Produkt eluiert, die noch 10 - 20 % p-Anteil enthalten. Das p-Produkt wurde weitgehend abgetrennt, indem der kristalline Rückstand aus der Flash-Chromatographie in so viel Diethylether gerührt wurde, daß 80 - 90 % in Lösung gingen. Das p-Produkt reicherte sich im ungelösten Rückstand an. Nach 2 - 3 sukzessiven Extraktionen war im $^1$H-NMR-Spektrum kein p-Anteil mehr zu erkennen. Ausbeute: 50 g (15 %) 3-(1-Oxo-2-(trifluoroacetamido-ethyl)phenylessigsäuremethylester (1b); Schmp. 74 - 75° C.

Analog wurden hergestellt:
3-(4-[1-Oxo-2-trifluoroacetamido-ethyl]phenyl)propionsäuremethylester (1c), Schmp. 155 - 157° C;
4-(1-Oxo-2-trifluoroacetamido-ethyl)phenoxyessigsäure (1d), Schmp. 145 - 148° C;
4-(1-Oxo-2-trifluoroacetamido-ethyl)phenol (1e), Schmp. 161 - 163° C.

Herstellung von 4-(1-Oxo-2-trifluoroacetamido-ethyl)phenoxyessigsäureethylester (1f)

In eine Lösung von 2,47 g 4-(1-Oxo-2-trifluoroacetamido-ethyl)phenol (1e) in 5 ml Dimethylsulfoxid wurden bei Raumtemperatur unter Rühren 1,06 g Natriumcarbonat oder 3,26 g Cäsiumcarbonat und nach 30 min 1,83 g Bromessigsäureethylester eingetragen. Ausfallender kristalliner Feststoff ließ die Mischung allmählich viskoser werden. Nach ca. 2 h erstarrte sie zu einem dicken, unrührbaren Kristallbrei.

Die kristalline Masse wurde in ca. 100 ml Wasser suspendiert, filtriert und mit Wasser nachgewaschen.

Enthält das Rohprodukt noch Ausgangsmaterial, so läßt sich dieses durch wiederholtes Waschen einer Essigsäureethylester-Lösung mit wässriger Natriumcarbonatlösung entfernen.
Ausbeute: 2,58 g (77 %) kristalliner Feststoff 1f; Schmp. 100 - 102° C.

Herstellung von 4-(2-Amino-1-oxo-ethyl)phenylessigsäurehydrochlorid (2a)

55,0 g 4-(1-Oxo-2-trifluoroacetamido-ethyl)phenylessigsäuremethylester (1a) wurden 2 h in 5 n Salzsäure unter Rückfluß gekocht. Die schwach getrübte Lösung wurde mit Aktivkohle gerührt, heiß filtriert, und im Eisbad der Kristallisation überlassen. Der kristalline Rückstand wurde filtriert, mit kaltem Wasser nachgewaschen und im Hochvakuum getrocknet (27,4 g). Die Mutterlauge wurde im Vakuum bis zur Trockene eingeengt und der Rückstand mit Isopropanol digeriert, filtriert und im Hochvakuum getrocknet (6,00 g). Beide Fraktionen waren von vergleichbarer Qualität. Gesamtausbeute: 33,4 g (80 %) kristalliner Feststoff 2a;

Schm. 259 °C (Z)

Analog wurden hergestellt:

3-(2-Amino-1-oxo-ethyl)phenylessigsäure-hydrochlorid (2b), Schmp. 192 °C (Z);

3-[4-(2-Amino-1-oxo-ethyl)phenyl]propionsäure-hydrochlorid, (2c), Schm. 192 - 195 °C (Z);

4-(2-Amino-1-oxo-ethyl)phenoxyessigsäure-hydrochlorid (2d), Schmp. 220 °C (Z);

4-(2-Amino-1-hydroxy-ethyl)phenylessigsäure-hydrochlorid (4a), Schmp. 202 - 203 °C;

3-(2-Amino-1-hydroxy-ethyl)phenylessigsäure-hydrochlorid (4b), $R_f$ (Fließmittel C) = 0,39;

3-[4-(2-Amino-1-hydroxy-ethyl)phenyl]propionsäure-hydrochlorid (4c), Schmp. 185 - 188 °C,

Herstellung von 4-(1-Hydroxy-2-trifluoroacetamidoethyl)phenylessigsäuremethylester (3a)

Zu einer Lösung von 36,0 g 4-(1-Oxo-2-trifluoroacetamido-ethyl)phenylessigsäuremethylester (1a) in 400 ml Isopropanol wurden bei Raumtemperatur unter Rühren 2,30 g Natriumborhydrid portionsweise innerhalb von 20 min eingetragen. Das Reaktionsgemisch wurde 3 h nachgerührt, bis die Gasentwicklung vollständig zum Erliegen gekommen war.

Das Lösungsmittel wurde im Vakuum vollständig abgezogen, der schaumige Rückstand in 100 ml Wasser aufgenommen und 2 mal mit je 300 ml Diethylether extrahiert. Der Etherextrakt wurde getrocknet, filtriert, im Vakuum bis zur Trockene eingeengt und der so gewonnene Rückstand im Hochvakuum getrocknet. Ausbeute: 29,0 g (80 %) schmieriger Feststoff 3a; $R_f$ (Fließmittel B) = 0,78.

Analog wurden hergestellt:

3-(1-Hydroxy-2-trifluoroacetamido-ethyl)phenylessigsäuremethylester (3b), Schmp. 95 - 97 °C;

3-(4-(1-Hydroxy-2-trifluoroacetamido-ethyl)phenyl)propionsäuremethylester (3c), Schmp. 178 °C (Z).

Herstellung von 3-(4-[1-Hydroxy-2-(trifluoroacetamido)ethyl]phenyl)propionsäuremethylester (3c)

Eine Lösung von 20,0 g 3-[4-(2-Amino-1-oxo-ethyl)phenyl]propionsäure-hydrochlorid (2c) in Methanol wurde nach Zugabe von 1,00 g Palladium auf Aktivkohle 2 h bei Normaldruck und Raumtemperatur hydriert. Der Katalysator wurde abfiltriert, die Mutterlauge im Vakuum bis zur Trockene eingeengt und der Rückstand im Hochvakuum getrocknet. Ausbeute: 20,0 g (99 %) kristalliner Feststoff 3c; Schmp. 178 °C (Z).

Analog wurden hergestellt:

4-(1-Hydroxy-2-trifluoroacetamido-ethyl)phenylessigsäuremethylester (3a), $R_f$ (Fließmittel B) = 0,78;

3-(1-Hydroxy-2-trifluoroacetamido-ethyl)phenylessigsäuremethylester (3b), Schmp. 95 - 97 °C;

4-(2-Amino-1-hydroxy-ethyl)phenylessigsäure-hydrochlorid (4a), Schmp. 202 - 203 °C;

4-(2-Amino-1-hydroxy-ethyl)phenoxyessigsäure-hydrochlorid (4d), Schmp. 190 °C (Z).

Herstellung von 4-(1-Hydroxy-2-phthalimidoethyl)phenylessigsäure (5a)

Zu dem in 150 ml Wasser fast vollständig gelösten Gemisch aus 10,4 g 4-(2-Amino-1-hydroxy-ethyl)-phenylessigsäure-hydrochlorid (4a) und 5,30 g Natriumcarbonat wurden innerhalb von einer Stunde bei Raumtemperatur 9,85 g Phthalimid-carbonsäureethylester unter Rühren portionsweise zugegeben. Nach weiteren 3 Stunden Rühren wurde die Lösung mit Natriumbicarbonat auf pH 8 eingestellt, mit Essigsäureethylester gewaschen, mit Natriumbisulfat auf pH 3 angesäuert und unter Eiskühlung der Kristallisation überlassen. Ausbeute: 9,80 g (67 %) kristalliner Feststoff 5a; Schmp. 205 - 208 °C.

Analog wurden hergestellt:

3-(1-Hydroxy-2-phthalimidoethyl)phenylessigsäure (5b), Schmp. 107 - 109 °C;

3-[4-(1-Hydroxy-2-phthalimidoethyl)phenyl]propionsäure (5c), $R_f$ (Fließmittel B) = 0,63;

N-Phthaloyl-octopamin (5d), Schmp. 207 - 209 °C.

Herstellung von 4-(1-Methoxy-2-phthalimidoethyl)phenylessigsäuremethylester (7a)

9,25 g 4-(1-Hydroxy-2-phthalimidoethyl)phenylessigsäure (5a) wurden in ca. 200 ml frisch destilliertem, säure- und wasserfreiem Dichlormethan suspendiert, auf 0 °C gekühlt und unter $N_2$-Schutz tropfenweise mit trockener etherischer Diazomethanlösung versetzt, bis eine schwache Gelbfärbung bestehen blieb. Der suspendierte Feststoff ging dabei als 4-(1-Hydroxy-2-phthalimidoethyl)phenylessigsäuremethylester (6a)

merklich in Lösung. Nach 15 min Rühren bei 0°C wurden unter $N_2$-Schutz 0,1 ml Zinn(IV)-chlorid zugegeben, wobei eine schlagartige Entfärbung der Suspension eintritt. Während der erneuten tropfenweise Zugabe von etherischer Diazomethanlösung ging auch der restliche suspendierte Feststoff in Lösung. Sobald die Gelbfärbung der Reaktionslösung bestehen blieb, wurde die Zugabe an Diazomethanlösung eingestellt, noch 30 min bei Raumtemperatur gerührt und die Lösung dann mit wenigen Tropfen Eisessig entfärbt. Geringe Mengen einer polymeren Verunreinigung wurden abfiltriert und das Filtrat im Vakuum bis zur Trockene eingeengt. Der kristalline Rückstand (9,30 g) wurde in 50 ml Isopropanol/Essigsäureethylester heiß gelöst und in 900 ml eiskaltes Pentan eingetropft. Ausbeute: 9,20 g (92 %) kristalliner Feststoff 7a; Schmp. 106 - 108°C.

Analog wurden hergestellt:
3-(1-Methoxy-2-phthalimidoethyl)phenylessigsäuremethylester (7b), Schmp. 76 - 78°C;
3-[4-(1-Methoxy-2-phthalimidoethyl)phenyl]propionsäuremethylester (7c), Schmp. 125 - 127°C;
4-[1-Methoxy-2-(N-phenylsulfonyl-N-methylamino) ethyl]phenoxyessigsäuremethylester (14), $R_f$ (Fließmittel A) = 0,68.

Herstellung von 3-[4-(1-Methoxy-2-phthalimidoethyl)phenyl]propionsäuremethylester (7c)

Eine 0°C kalte Lösung von 4,60 g 3-[4-(1-Hydroxy-2-phthalimidoethyl)phenyl]propionsäure (5c) und 3,36 ml Methyljodid in 45 ml Dimethylformamid wurde innerhalb von 1 h portionsweise mit 6,24 g Silber-II-oxid versetzt. Das Gemisch wurde im Eisbad und anschließend bei Raumtemperatur über Nacht gerührt. 3 Tage lang wurde täglich nach dem Abkühlen auf 0°C je ein equivalent Methyljodid und Silber-II-oxid zugegeben.

Das Reaktionsgemisch wurde in 500 ml Wasser gegossen und 2 mal mit je 250 ml Diethylether extrahiert. Der Etherextrakt wurde getrocknet, filtriert und im Vakuum bis zur Trockene eingeengt.

Der ölige Rückstand (ca. 5 g) wurde durch Flash-Chromatographie (Kieselgel 60, mesh 230 (Merck 7729) gereinigt. Mit 10 % Essigsäureethylester/90 % Cyclohexan ließen sich 3,90 g (79 %) des gewünschten Produktes 7c, Schmp. 125 - 127°C, eluieren. Bei 50 % Essigsäureethylester/50 % Cyclohexan eluierten noch 200 mg (4 %) 3-[4-(1-Hydroxy-2-phthalimidoethyl)phenyl]propionsäuremethylester (6c).

Analog wurden hergestellt:
4-(1-Methoxy-2-phthalimidoethyl)phenoxyessigsäureethylester (7d), Schmp. 100 - 105°C;
4-(1-Ethoxy-2-phthalimidoethyl)phenoxyessigsäureethylester (7e), $R_f$ (Fließmittel A) = 0,79;
4-[1-Methoxy, 2-(N-phenylsulfonyl-N-methylamino)ethyl]phenoxyessigsäuremethylester (14), $R_f$ (Fließmittel A) = 0,68.

Herstellung von 4-(1-Methoxy-2-aminoethyl)phenoxyessigsäure (8d)

Ein Gemisch aus 8,30 g 4-(1-Methoxy-2-phthalimidoethyl)phenoxyessigsäureethylester (7d) und 83 ml 25 %ige wässrige Kaliumhydroxidlösung wurde 6 h bei 100 - 110°C gerührt.

Die wässrige Lösung wurde mit Salzsäure auf pH 3 eingestellt, 2 mal mit je 100 ml Essigsäurethylester oder Diethylether extrahiert, mit Natriumbicarbonatlösung auf pH 7 eingestellt und im Eisbad der Kristallisation überlassen. Nach dem Filtrieren und Waschen wurde der kristalline Rückstand im Hochvakuum getrocknet. Ausbeute: 2,36 g (49 %) kristalliner Feststoff 8d; Schmp. 107 - 110°C. Die Mutterlauge enthielt noch Produkt.

Alternativ kann das wäßrige Hydrolysat nach Neutralisation mit Salzsäure, ohne weitere Aufarbeitung und Isolierung des Produktes, direkt der Sulfonierung zugeführt werden (vgl. Beispiel 9d).

Herstellung von N-Phenylsulfonyl-octopamin (10a)

In eine Lösung von 18,9 g Octopamin-hydrochlorid und 32,8 g Natriumacetat in 200 ml Ethanol wurden unter Rühren bei Raumtemperatur 17,6 g Benzolsulfonylchlorid innerhalb von 30 min eingetropft. Nach weiteren 30 min Rühren wurde das Reaktionsgemisch in 2 l Wasser eingegossen. Der ausfallende kristalline Feststoff wurde abfiltriert, nachgewaschen, getrocknet und aus ca. 300 ml Methanol umkristallisiert (9,95 g). Aus der methanolischen Mutterlauge ließen sich weitere 6,95 g reines Produkt gewinnen. Ausbeute: 16,9 g (58 %) kristalliner Feststoff 10a; Schmp. 166-170°C.

Analog wurden hergestellt:

N-(4-Chlor-phenylsulfonyl)-octopamin (10b), Schmp. 146-148° C;
N-(4-Fluor-phenylsulfonyl)-octopamin (10c), Schmp. 118-122° C.

## Herstellung der erfindungsgemäßen Verbindungen

### Beispiel 1

Herstellung von 4-(1-Hydroxy-2-phthalimidoethyl)-phenoxyessigsäureethylester (6d)

In eine Lösung von 14,1 g N-Phthaloyl-octopamin (5d) in 30 ml Dimethylsulfoxid wurden bei Raumtemperatur unter Rühren 16,3 g Cäsiumcarbonat eingetragen. Nach 10 min Rühren wurden 12,5 g Bromessigsäureethylester innerhalb von 30 min zugetropft. Die Reaktionsmischung wurde 4 h bei Raumtemperatur gerührt, mit Wasser auf ca. 500 ml verdünnt und 2 mal mit je 100 ml Essigsäureethylester extrahiert. Die Essigsäureethylester-Lösung wurde getrocknet, filtriert und im Vakuum bis zur Trockene eingeengt. Der ölige Rückstand (18,8 g) wurde mit 200 ml Diethylether digeriert, wobei er kristallisierte. Ausbeute: 13,9 g (75 %) kristalliner Feststoff 6d; Schmp. 95 - 96° C.

Analog wurden hergestellt:

4-(1-Hydroxy-2-phenylsulfonamido-ethyl)phenoxyessigsäureethylester (11a), $R_f$ (Fließmittel B) = 0,68;
4-[1-Hydroxy-2-(4-chlorphenylsulfonamido)ethyl]phenoxyessigsäureethylester (11b), $R_f$ (Fließmittel B) = 0,76;
4-[1-Hydroxy-2-(4-fluorphenylsulfonamido)ethyl]phenoxyessigsäureethylester (11c), $R_f$ (Fließmittel B) = 0,71.

### Beispiel 2

Herstellung von 4-(1-Methoxy-2-phenylsulfonamido-ethyl)phenoxyessigsäure (9d)

Eine Lösung von 4,89 g 4-(1-Methoxy-2-aminoethyl)phenoxyessigsäure (8d) in 100 ml Wasser oder das neutralisierte wässrige Hydrolysat von 8,32 g 4-(1-Methoxy-2-phthalimidoethyl)phenoxyessigsäureethylester (7d) (vgl. Anmerkung zu Beispiel 8d) wurde, mit 4,14 g Pottasche gepuffert, innerhalb von 30 min bei Raumtemperatur mit einer Lösung von 4,21 g Benzolsulfonsäurechlorid in 5 ml Dimethylformamid versetzt und das Reaktionsgemisch weitere 2 h gerührt.

Das wässrige Gemisch wurde mit 100 ml Essigsäureethylester gewaschen, mit Natriumbisulfat auf pH 3 angesäuert und 2 mal mit je 150 ml Diethylether extrahiert. Die etherische Lösung wurde getrocknet, filtriert und im Vakuum bis zur Trockene eingeengt.

Der ölige Rückstand (ca. 6 g) wurde durch Flash-Chromatographie (Kieselgel 60, mesh 230 (Merck 7729) gereinigt. Das gewünschte Produkt eluiert zwischen 40 % Essigsäureethylester/60 % Cyclohexan und 100 % Essigsäureethylester. Ausbeute: 3,66 g (46 %) 9d als Öl; $R_f$ (Fließmittel B) = 0,40; Schmp. (Ammoniumsalz): 135° C (Z).

Analog wurden hergestellt:

4-(1-Methoxy-2-phenylsulfonamido-ethyl)phenylessigsäure (9a), Schmp. 121 - 122° C;
3-(1-Methoxy-2-phenylsulfonamido-ethyl)phenylessigsäure (9b), amorph;
3-(4-[1-Methoxy-2-phenylsulfonamido-ethyl]phenyl)propionsäure (9c), Schmp. (Kaliumsalz): 145° C (Z);
4-[1-Methoxy-2-(4-chlor-phenylsulfonamido)ethyl]phenylessigsäure (9e), Schmp. 130 - 131° C;
3-[1-Methoxy-2-(4-chlor-phenylsulfonamido)ethyl]phenylessigsäure (9f), amorph;
3-(4-[1-Methoxy-2-(4-chlor-phenylsulfonamido)ethyl]phenyl)propionsäure (9g), Schmp. 149° C (Z);
4-[1-Methoxy-2-(4-chlor-phenylsulfonamido)ethyl]phenoxyessigsäure (9h), Schmp. 135° C (Z);
4-(1-Ethoxy-2-phenylsulfonamido-ethyl)phenoxyessigsäure (9i), Schmp. 130° C (Z).
4-(1-Oxo-2-phenylsulfonamido-ethyl)phenylessigsäure (16a), Schmp. 156 - 160° C;
3-(1-Oxo-2-phenylsulfonamido-ethyl)phenylessigsäure (16b), Schmp. 135 - 137° C;
4-(1-Oxo-2-4-chlor-phenylsulfonamido-ethyl)phenylessigsäure (16d), Schmp. 188 - 191° C;
3-(1-Oxo-2-4-chlor-phenylsulfonamido-ethyl)phenylessigsäure (16e), Schmp. 174° C (Z);

3-(4-[1-Oxo-2-phenylsulfonamido-ethyl]phenyl)propionsäure (16c), Schmp. 150 - 155° C;
3-(4-[1-Oxo-2-(4-chlor-phenylsulfonamido)-ethyl]phenyl)propionsäure (16f), Schmp. 196 - 198° C
3-(4-[1-Oxo-2-(4-methyl-phenylsulfonamido)ethyl]phenyl)propionsäure (16g), Schmp. 166 - 167° C;
4-[1-Hydroxy-2-(phenylsulfonamido)ethyl]phenylessigsäure (17a), Schmp. 151 - 155° C;
3-(1-Hydroxy-2-phenylsulfonamido-ethyl)phenylessigsäure (17b), Schmp. (Ammoniumsalz): 59 - 65° C;
4-[1-Hydroxy-2-(4-chlor-phenylsulfonamido)ethyl]phenylessigsäure (17d), Schmp. 159 - 164° C;
3-[1-Hydroxy-2-(4-chlor-phenylsulfonamido)ethyl]phenylessigsäure (17e), Schmp. 96 - 102° C;
3-(4-[1-Hydroxy-2-phenylsulfonamido-ethyl]phenyl)-propionsäure (17c), Schmp. 128 - 133° C;
3-(4-[1-Hydroxy-2-(4-chlorphenylsulfonamido)ethyl]phenyl)-propionsäure (17f), Schmp. 143 - 150° C;
4-(1-Hydroxy-2-phenylsulfonamido-ethyl)phenoxyessigsäure (12a), Schmp. (Kaliumsalz): 180° C (Z);
4-[1-Hydroxy-2-(4-chlorphenylsulfonamido)ethyl]phenoxyessigsäure (12b), Schmp. 113 - 116° C;
4-[1-Hydroxy-2-(4-fluorphenylsulfonamido)ethyl]phenoxyessigsäure (12c), Schmp. 114 - 117° C.

Beispiel 3

Herstellung von 4-[1-Hydroxy-2-(4-chlorphenylsulfonamido)ethyl]phenoxyessigsäure (12b)

10,2 g 4-[1-Hydroxy-2-(4-chlorphenylsulfonamido)ethyl]phenoxyessigsäureethylester (11b) (Darstellung analog Beispiel 1f) wurden bei Raumtemperatur 1 h in 25 ml 1 n NaOH gerührt. Die Lösung wurde mit Essigsäureethylester gewaschen, mit Natriumbisulfat auf pH 3 angesäuert und 2 mal mit je 50 ml Diethylether extrahiert. Die etherische Lösung wurde getrocknet, filtriert und im Vakuum bis zur Trockene eingeengt. Der schmierige Rückstand wurde durch digerieren mit ca. 200 ml Diisopropylether zur Kristallisation gebracht. Ausbeute: 5,67 g (59 %) kristalliner Feststoff 12b; Schmp. 113 - 116° C.

Analog wurden hergestellt:
4-(1-Hydroxy-2-phenylsulfonamido-ethyl)phenoxyessigsäure (12a), Schmp. (Kaliumsalz): 180° C (Z);
4-[1-Hydroxy-2-(4-fluorphenylsulfonamido)ethyl]phenoxyessigsäure (12c), Schmp. 114 - 117° C;
4-[1-Methoxy-2-(N-phenylsulfonyl, N-methylamino)ethyl]phenoxyessigsäure (15), Schmp. (Ammoniumsalz): 60 - 65° C.

Beispiel 4

Herstellung von 4-[1-Acetoxy-2-(4-chlorphenylsulfonamido)ethyl]phenoxyessigsäure (13b)

2 g 4-[1-Hydroxy-2-(4-chlorphenylsulfonamido)ethyl]phenoxyessigsäure (12b) wurden in einem Gemisch aus 10 ml Eisessig und 10 ml Acetanhydrid gelöst. Nach Zugabe von 100 mg N,N-Dimethylaminopyridin wurde die Reaktionslösung 8 h bei 50° C gerührt.

Die Lösung wurde im Vakuum vollständig eingedampft, der Rückstand in wenig Essigsäureethylester gelöst, mit Wasser gewaschen, getrocknet, filtriert, das Filtrat auf ein Volumen von ca. 10 ml eingeengt und langsam in 200 ml eiskalten Petrolether eingetropft. Der ausfallende kristalline Feststoff wurde abfiltriert und im Hochvakuum getrocknet. Ausbeute: 1,25 g (57 %) kristalliner Feststoff 13b; Schmp. 137 - 140° C.

Analog wurde hergestellt:
4-(1-Acetoxy-2-phenylsulfonamido-ethyl)phenoxyessigsäure (13a), Schmp. 216° C (Z).

Zur Untersuchung der pharmakodynamischen Eigenschaften der erfindungsgemäßen Produkte wurden folgende Methoden verwendet:

1. Hemmung der durch Thromboxan-$A_2$-Analog (U 46619) induzierten Aggregation von Thrombozyten des Menschen, des Kaninchens oder des Hundes in vitro

Thrombozytenreiches Plasma wird durch Zentrifugation (160 x, 16 min Dauer bei 4° C) aus venösem Citratblut gewonnen. Die photometrische Messung der Thrombozytenaggregation erfolgt durch Zusatz von $MgCl_2$ (Endkonzentration 10 mmol/l) und von U 46619 (Endkonzentration 5 x $10^{-7}$ mol/l) im Born-Aggregometer Mk 3. Als Aggregationsmaß findet die maximale Extinktionsänderung/sec Verwendung. Die Prüfung der aggregationshemmenden Wirksamkeit der Substanzen wird nach einer Inkubationszeit von 10

min vorgenommen.

Prozentuale Hemmung der Thrombozytenaggregation in vitro durch einige erfindungsgemäße Verbindungen bei 0,1 mg/l

| Verbindung | thrombozytenreiches Plasma | | |
|---|---|---|---|
| | Mensch | Hund | Kaninchen |
| 9a | 34 % | - | - |
| 9b | 32 % | - | - |
| 9d | 26 % | - | - |
| 9e | 70 % | 65 % | 41 % |
| 9f | 19 % | - | - |
| 9h | 64 % | - | - |
| 16a | 58 % | - | - |
| 16c | 87 % | - | - |
| 16d | 9 % | - | - |
| 16f | 96 % | - | - |
| 16g | 76 % | 81 % | 16 % |
| 17c | 28 % | — | — |
| 17d | 12 % | — | — |

2. Hemmung der durch Thromboxan-$A_2$-Analog (U 46619) induzierten Aggregation von Thrombozyten des Kaninchens und des Hundes ex vivo

Die Substanzen werden Gruppen von 12 bis 16 Kaninchen (weiße Neuseeländer, 2.5 bis 3.2 kg) bzw. 3 bis 5 Beagle-Hunden intravenös oder oral appliziert. Zu verschiedenen Zeiten nach intravenöser Applikation oder oraler Applikation wird den Tieren Blut entnommen und durch Zentrifugation thrombozytenreiches Plasma gewonnen. Die Messung der Aggregation nach Zusatz von U 46619 erfolgt wie unter 1. angegeben. Als ED 50 % wird die Dosis bestimmt, welche die durch das Thromboxan-$A_2$-Analog induzierte Thrombozytenaggregation um 50 % hemmt.

3. In-vivo-Hemmung der Thrombozytenaggregation am narkotisierten Kaninchen

Zur Prüfung der thrombozytenaggregationshemmenden Wirkung der Substanzen wird beim narkotisierten Kaninchen nach einer Laparotomie die Aorta abdominalis einige Zentimeter schonend freipräpariert und mit einem elektromagnetischen Durchflußmeßkopf versehen. Ca. 1 cm distal vom Flußmeßkopf wird das Gefäß mit Hilfe einer Klemme mechanisch geschädigt. Durch eine definierte Verengung des Gefäßes mit einem Konstriktor mit geeignetem Durchmesser werden zyklisch wiederkehrende Blutflußveränderungen erzeugt, welche durch die Aggregation und nachfolgende spontane Loslösung von Thrombozyten im Bereich der Stenose entstehen. Die Verringerung der Anzahl und Amplitude dieser zyklischen Blutflußvariationen dient als Maß für die Wirksamkeit der Substanzen.

## Ansprüche

1. Sulfonamid-Derivate der Formel I

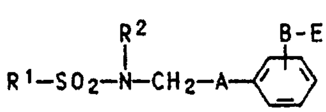

I

in der
$R^1$ eine $C_{1-4}$-Alkyl- oder Phenylgruppe bedeutet, wobei die Phenylgruppe durch Halogenatome, Trifluoromethyl- oder $C_{1-4}$-Alkylgruppen substituiert sein kann,
$R^2$ ein Wasserstoffatom oder eine $C_{1-4}$-Alkylgruppe bedeutet,
A für eines der Brückenglieder $CHOR^3$, C=O, C=S oder $C=N-OR^4$ steht, wobei $R^3$ ein Wasserstoffatom, eine $C_{1-4}$-Alkyl-, Phenyl-, Benzyl- oder $C_{1-4}$-Acylgruppe und $R^4$ ein Wasserstoffatom oder eine Methylgruppe bedeutet,
B eine m- oder p-ständige Methylen-, Ethylen- oder, wenn A nicht für eine Carbonylgruppe steht, auch eine Methylenoxy-Gruppe bedeuten kann, wobei das Sauerstoffatom an den Aromaten gebunden ist,
E einen Carboxyl-, Phosphonsäure- oder Sulfonsäurerest, deren physiologisch unbedenkliche Salze, Ester mit $C_{1-4}$-Alkoholen und Amide, sowie den Tetrazol-5-yl-Rest bedeuten kann.

2. Ein Sulfonamid-Derivat nach Anspruch 1 aus der Gruppe:
4-(1-Oxo-2-phenylsulfonamido-ethyl)phenylessigsäure
3-(1-Oxo-2-phenylsulfonamido-ethyl)phenylessigsäure
4-[1-Oxo-2-(4-chlor-phenylsulfonamido)ethyl]phenylessigsäure
3-[1-Oxo-2-(4-chlor-phenylsulfonamido)ethyl]phenylessigsäure
3-(4-[1-Oxo-2-(phenylsulfonamido)ethyl]phenyl)propionsäure
3-(4-[1-Thiono-2-(phenylsulfonamido)ethyl]phenyl)propionsäure
3-(4-[1-Oximino-2-(phenylsulfonamido)ethyl]phenyl)propionsäure
3-(4-[1-Methoximino-2-(phenylsulfonamido)ethyl]phenyl)propionsäure
3-(4-[1-Oxo-2-(4-methyl-phenylsulfonamido)-ethyl]phenyl)propionsäure
3-(4-[1-Oxo-2-(3,4-dimethyl-phenylsulfonamido)-ethyl]phenyl)propionsäure
3-(4-[1-Oxo-2-(2,4,6-trimethyl-phenylsulfonamido)-ethyl]phenyl)propionsäure
3-(4-[1-Oxo-2-(4-ethyl-phenylsulfonamido)-ethyl]phenyl)propionsäure
3-(4-[1-Oxo-2-(4-methoxy-phenylsulfonamido)-ethyl]phenyl)propionsäure
3-(4-[1-Oxo-2-(4-trifluoromethyl-phenylsulfonamido)-ethyl]phenyl)propionsäure
3-(4-[1-Oxo-2-(4-fluor-phenylsulfonamido)-ethyl]phenyl)propionsäure
3-(4-[1-Oxo-2-(2-chlor-phenylsulfonamido)-ethyl]phenyl)propionsäure
3-(4-[1-Oxo-2-(2,6-dichlor-phenylsulfonamido)-ethyl]phenyl)propionsäure
3-(4-[1-Oxo-2-(4-chlor-phenylsulfonamido)-ethyl]phenyl)propionsäure
3-(4-[1-Thiono-2-(4-chlor-phenylsulfonamido)-ethyl]phenyl)propionsäure
3-(4-[1-Oximino-2-(4-chlor-phenylsulfonamido)-ethyl]phenyl)propionsäure
3-(4-[1-Methoximino-2-(4-chlor-phenylsulfonamido)-ethyl]phenyl)propionsäure
4-(1-Hydroxy-2-phenylsulfonamido-ethyl)phenylessigsäure
3-(1-Hydroxy-2-phenylsulfonamido-ethyl)phenylessigsäure
4-[1-Hydroxy-2-(4-chlor-phenylsulfonamido)ethyl]phenylessigsäure
3-[1-Hydroxy-2-(4-chlor-phenylsulfonamido)ethyl]phenylessigsäure
3-(4-[1-Hydroxy-2-(phenylsulfonamido)ethyl]phenyl-propionsäure
3-(4-[1-Hydroxy-2-(4-chlor-phenylsulfonamido-ethyl]phenylpropionsäure
4-(1-Hydroxy-2-phenylsulfonamido-ethyl)phenoxyessigsäure
4-[1-Hydroxy-2-(4-chlor-phenylsulfonamidoethyl]phenoxyessigsäure
4-[1-Hydroxy-2-(4-fluor-phenylsulfonamidoethyl]phenoxyessigsäure
4-[1-Hydroxy-2-(4-chlor-phenylsulfonamidoethyl]phenoxymethansulfonsäure
4-[1-Hydroxy-2-(4-chlor-phenylsulfonamidoethyl]phenoxymethanphosphonsäure
4-(1-Methoxy-2-phenylsulfonamido-ethyl)phenylessigsäure
3-[1-Methoxy-2-phenylsulfonamido-ethyl)phenylessigsäure
4-[1-Methoxy-2-(4-chlor-phenylsulfonamido)ethyl]phenylessigsäure
(+)-4-[1-Methoxy-2-(4-chlor-phenylsulfonamido)ethyl]phenylessigsäure
(-)-4-[1-Methoxy-2-(4-chlor-phenylsulfonamido)ethyl]phenylessigsäure
3-[1-Methoxy-2-(4-chlor-phenylsulfonamido)ethyl]phenylessigsäure
3-(4-[1-Methoxy-2-phenylsulfonamido-ethyl]phenyl)propionsäure
3-(4-[1-Methoxy-2-(4-chlor-phenylsulfonamido)ethyl]phenyl)propionsäure
4-(1-Methoxy-2-phenylsulfonamido-ethyl)phenoxyessigsäure
4-[1-Methoxy-2-(4-fluor-phenylsulfonamidoethyl]phenylessigsäure
4-[1-Methoxy-2-(4-chlor-phenylsulfonamidoethyl]phenoxyessigsäure
(+)-4-[1-Methoxy-2-(4-chlor-phenylsulfonamido)ethyl]phenoxyessigsäure
(-)-4-[1-Methoxy-2-(4-chlor-phenylsulfonamido)ethyl]phenoxyessigsäure
4-(1-Acetoxy-2-phenylsulfonamido-ethyl)phenoxyessigsäure

4-[1-Acetoxy-2-(4-chlor-phenylsulfonamido)ethyl]phenoxyessigsäure
4-(1-Ethoxy-2-phenylsulfonamido-ethyl)phenoxyessigsäure
4-[1-Methoxy-2-(N-phenylsulfonyl, N-methylamino)ethyl]phenoxyessigsäure

3. Arzneimittel zur parenteralen Anwendung, das pro Einzeldosis 0,1 bis 10 mg einer Verbindung nach Anspruch 1 oder 2 als Wirkstoff neben üblichen galenischen Hilfsmitteln enthält.

4. Arzneimittel zur oralen Anwendung, das pro Einzeldosis 1 bis 100 mg einer Verbindung nach Anspruch 1 oder 2 als Wirkstoff neben üblichen galenischen Hilfsmitteln enthält.

5. Verwendung der Verbindungen der Formel I gemäß Anspruch 1 zur Herstellung von Arzneimitteln zur Behandlung und zur Prophylaxe thrombo-embolischer Erkrankungen, zur Prophylaxe der Arteriosklerose und zur Metastasenprophylaxe.

6. Verfahren zur Herstellung der Verbindungen der Formel I gemäß Anspruch 1, dadurch gekennzeichnet, daß man

a) Verbindungen der Formel II

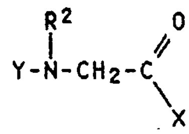

II

in der
$R^2$ ein Wasserstoffatom oder eine $C_{1-4}$-Alkylgruppe bedeutet,
X eine Abgangsgruppe darstellt und
Y für eine hydrolytisch oder hydrogenolytisch abspaltbare Schutzgruppe oder für den Rest $R^1$ mit der in Formel I beschriebenen Bedeutung steht, umsetzt mit einer Verbindung der Formel III

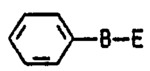

III,

in der B und E die für Formel I beschriebenen Bedeutungen besitzen, von den so erhaltenen Verbindungen die Schutzgruppe nach üblichen Methoden abspaltet und durch den Rest $R_1$-$SO_2$-, mit der in Formel I beschriebenen Bedeutung, ersetzt und gegebenenfalls die Carbonylgruppe zum Alkohol reduziert und derivatisiert, wobei die vorstehend genannten Reaktionen auch in jeder anderen sinnvollen Reihenfolge durchgeführt werden oder teilweise entfallen können;

b) Octopamin mit Verbindungen der Formel IV

$R^1$-$S0_2$-X IV

in der
$R^1$ die in Formel I beschreibene Bedeutung zukommt und X eine Abgangsgruppe darstellt, umsetzt und die so erhaltenen Verbindungen am phenolischen Sauerstoff mit einer Verbindung der Formel V

X-$CH_2$-E-Y V

in der
X eine Abgangsgruppe darstellt,
E die in Formel I beschriebene Bedeutung besitzt und
Y gegebenenfalls für eine hydrolytisch oder hydrogenolytisch abspaltbare Schutzgruppe steht,
alkyliert und gegebenenfalls die aliphatische Hydroxy-Gruppe derivatisiert, wobei die vorstehend genannten Reaktionen auch in jeder anderen sinnvollen Reihenfolge durchgeführt werden oder teilweise entfallen können.